# EUROPÄISCHE PATENTSCHRIFT

(11) **EP 4 547 163 B1**
(45) Veröffentlichungstag und Bekanntmachung des Hinweises auf die Patenterteilung: **22.04.2026**
(21) Anmeldenummer: 23732154.2
(22) Anmeldetag: 16.06.2023
(51) Int. Cl.: A61F 2/46

(54) **CHIRURGISCHES INSTRUMENT**
SURGICAL INSTRUMENT
INSTRUMENT CHIRURGICAL

(30) Priorität: 30.06.2022 DE 102022116409
(43) Veröffentlichungstag der Anmeldung: 07.05.2025
(73) Patentinhaber: Endocon GmbH, 69257 Wiesenbach (DE)
(72) Erfinder: NOTARBARTOLO, Klaus, 69257 Wiesenbach (DE)
(74) Vertreter: Reiser & Partner Patentanwälte mbB
(86) Internationale Anmeldenummer: PCT/EP2023/066236
(87) Internationale Veröffentlichungsnummer: WO 2024/002719

(56) Entgegenhaltungen:
- EP-B1- 0 910 317
- US-A- 4 651 833
- US-A- 5 485 887
- US-B2- 8 602 124

## Beschreibung

Die Erfindung betrifft ein chirurgisches Instrument zur Revision von Prothesen, umfassend ein Handstück, in welchem ein Zylinder angeordnet ist, wobei dem Zylinder ein Kolbenelement zugeordnet ist sowie ein Schaltelement und ein Meißelwerkzeug, wobei das Meißelwerkzeug an einem Ende des Handstücks axial beweglich gelagert angeordnet ist, wobei das Meißelwerkzeug verliersicher am Handstück befestigt ist, wobei das Kolbenelement in dem Zylinder axial beweglich angeordnet ist, wobei das Kolbenelement mittels eines Fluids in Bewegung versetzbar ist.

Aus der EP 0 910 317 B1 ist ein chirurgisches Instrument zur Revision von Prothesen bekannt, bei dem ein Kolbenelement pneumatisch in einem Zylinder hinund herbewegt wird, wobei das Kolbenelement einen Schlag auf ein in dem Gehäuse axial gelagertes Meißelwerkzeug ausübt. Mit einem solchen chirurgischen Instrument lässt sich ein Meißelwerkzeug in einen Zwischenraum zwischen dem der Prothese zugeordneten Prothesenschaft und der in einen Knochen eingebrachten Ausnehmung eintreiben. Der Zwischenraum wird auch als Prothesenschaftzwischenraum bezeichnet.

US 4,651,833 A zeigt ein pneumatisches Schlagwerkzeug, bei dem ein Kolben in einer Kammer eines Zylinders hin- und herbewegbar ist. Das Schlagwerkzeug, das in einen Knochen eindringen soll, ist im vorderen Teil des Zylinders angebracht, und der Kolben schlägt gegen diesen vorderen Teil als Reaktion auf die Zufuhr von Druckluft gegen seine hintere Endfläche.

Das Meißelwerkzeug ist üblicherweise länglich geformt und flexibel. Eine solche Ausgestaltung begünstigt, dass das Meißelwerkzeug sehr weit in den Prothesenschaftzwischenraum eindringen kann. Dabei ist aber nachteilig, dass sich das Meißelwerkzeug in dem Prothesenschaftzwischenraum verklemmen kann. Das Lösen des Meißelwerkzeugs ist dann nur mit größerem manuellem Aufwand möglich. Beispielsweise ist es bekannt, einen Schlitzhammer an der Unterseite des Handstücks auf das Meißelwerkzeug aufzusetzen und durch kontrollierte Hammerschläge eines zweiten Hammers das Meißelwerkzeug zu lösen. Dabei besteht aber eine Verletzungsgefahr für die der Prothese benachbarten Knochen und der Vorgang ist für den Chirurgen zeitintensiv und nicht ergonomisch.

Der Erfindung liegt die Aufgabe zugrunde, ein chirurgisches Instrument zur Revision von Prothesen anzugeben, welches eine verbesserte Handhabbarkeit aufweist.

Die Aufgabe wir mit den Merkmalen von Anspruch 1 gelöst. Auf vorteilhafte Ausgestaltungen nehmen die Unteransprüche Bezug.

Das erfindungsgemäße chirurgische Instrument zur Revision von Prothesen umfasst ein Handstück, in welchem ein Zylinder angeordnet ist, wobei dem Zylinder ein Kolbenelement zugeordnet ist sowie ein Schaltelement und ein Meißelwerkzeug, wobei das Meißelwerkzeug an einem Ende des Handstücks axial beweglich gelagert angeordnet ist, wobei das Meißelwerkzeug verliersicher am Handstück befestigt ist, wobei das Kolbenelement in dem Zylinder axial beweglich angeordnet ist, wobei das Kolbenelement mittels eines Fluids in Bewegung versetzbar ist, wobei das Kolbenelement dazu eingerichtet ist, einen in Richtung des Meißelwerkzeugs gerichteten Impuls zum Eintreiben des Meißelwerkzeugs in einen der Prothese zugeordneten Prothesenschaftzwischenraum zu induzieren, wobei das Kolbenelement eingerichtet ist, einen dem Meißelwerkzeug entgegengerichteten Impuls zum Austreiben des Meißelwerkzeugs aus dem Prothesenschaftzwischenraum zu induzieren.

Durch den mittels eines Fluids und Kolbenelement kontrolliert erzeugbaren Impuls sowohl zum Eintreiben als auch zum Austreiben des Meißelwerkzeugs in einen bzw. aus dem der Prothese zugeordneten Prothesenschaftzwischenraum, kann eine Wiederholbarkeit des Impulses erreicht werden. Als Fluid werden Gase und Flüssigkeiten bezeichnet. Insbesondere ist das Kolbenelement pneumatisch oder hydraulisch in Bewegung versetzbar. Als mögliche Fluide können beispielsweise Druckluft oder Stickstoff verwendet werden. Prinzipiell ist denkbar, dass das Fluid eine Hydraulikflüssigkeit ist.

Der zum Austreiben des Meißelwerkzeugs erzeugbare Impuls kann genutzt werden, wenn sich das Meißelwerkzeug beispielsweise im Prothesenschaftzwischenraum verkantet und nicht mehr frei beweglich ist. Durch kontrolliert erzeugbare Impulse, auch für das Austreiben, wird der Arbeitsablauf standardisiert und vereinfacht, die Prozess- und Arbeitssicherheit wird erhöht und es kann eine gleichbleibende Qualität des gesamten Arbeitsablaufs erreicht werden. Der Zeitaufwand zur Revision von Prothesen wird reduziert, während gleichzeitig durch Reduktion möglicher Fehlbenutzungen die Haltbarkeit des chirurgischen Instruments gesteigert wird. Außerdem wird die Sicherheit des Patienten erhöht, da Schädigungen am Prothesenschaftzwischenraum und den umliegenden Körperpartien verringert werden.

Eine vorteilhafte Ausgestaltung der Erfindung sieht vor, dass das Kolbenelement mittels des Schaltelements wahlweise zum Eintreiben oder Austreiben des Meißelwerkzeugs einstellbar ist. Durch diese Maßnahme wird dem Nutzer eine einfache Auswahl des Betriebsmodus ermöglicht, wodurch die Handhabbarkeit des chirurgischen Instruments weiter erhöht wird.

Dem Zylinder ist erfindungsgemäß ein Impulsübertrager zugeordnet, wobei der Impulsübertrager der dem Meißelwerkzeug zugeordneten Stirnseite zugeordnet ist, so dass der Impulsübertrager zwischen Kolbenelement und Meißelwerkzeug angeordnet ist, wobei der Impulsübertrager dazu eingerichtet ist, einen Impuls von dem Kolbenelement auf das Meißelwerkzeug zu übertragen, wobei der Impulsübertrager die Stirnseite des Zylinders auf der dem Meißelwerkzeug zugewandten Seite abdichtet. Der Impulsübertrager ist dabei typischerweise ein qualitativ hochwertiges Bauteil und mit guten Führungseigenschaften versehen, um eine präzise Bewegung relativ zum Zylinder auszuführen. Weiterhin bietet der Impulsübertrager eine Aufnahme für das Meißelwerkzeug, so dass verschiedene Meißelwerkzeuge eingesetzt werden können ohne jedes Meißelwerkzeug mit hochwertigen Führungseigenschaften ausrüsten zu müssen. Dadurch werden die Kosten bei wechselnden Meißelwerkzeugen bei gleichbleibenden Qualitätsansprüchen reduziert.

Der Zylinder kann ein rohrförmiges Element sein, welches in das Handstück eingesetzt ist. Der Zylinder ist dabei vorzugsweise derart dimensioniert, dass das Kolbenelement in dem Zylinder entlang der Längsachse des Zylinders beweglich angeordnet ist. Dabei ist der Spalt zwischen Kolbenelement und Zylindermantel vorzugsweise derart ausgelegt, dass das Kolbenelement durch ein Fluid bewegt werden kann.

Dem Zylinder kann eine erste und eine zweite Staudruckkammer zugeordnet sein, wobei beide Staudruckkammern strömungsleitend mit dem Zylinder verbindbar sind, wobei dem Zylinder eine Fluidquelle zugeordnet ist. Die erste oder die zweite Staudruckkammer dient je nach Bewegungsrichtung des Kolbenelements unter anderem dazu, das vom Kolbenelement verdrängte Fluid aufzunehmen, wobei das verdrängte Fluid in der jeweiligen Staudruckkammer komprimiert wird. Vorzugsweise umgeben die erste und/oder die zweite Staudruckkammer den Zylinder zumindest teilweise entlang der Längsachse des Zylinders koaxial ringförmig. Die Fluidquelle kann zur Förderung eines Fluids genutzt werden und dient dem Kolbenelement als Antriebsmittel. Durch diese Maßnahme können beispielsweise gefährliche Flüssigkeiten als Antriebsmittel vermieden werden, wodurch die Prozess- und Arbeitssicherheit weiter erhöht werden kann. Die Ausgestaltung mit Staudruckkammern eignet sich insbesondere für komprimierbare, gasförmige Fluide als Arbeitsmedium. Bei Verwendung inkompressibler Fluide ist es erforderlich, dass die Staudruckkammern ein veränderliches Volumen aufweisen.

Der Zylinder kann auf der dem Meißelwerkzeug abgewandten Seite einen ersten Kanal umfassen, wobei der Zylinder vorzugsweise durch den ersten Kanal mit der Fluidquelle strömungsverbindbar ist. Auf der dem Meißelwerkzeug abgewandten Seite bedeutet im Sinne der Erfindung, dass der Kanal auf der dem Meißelwerkzeug abgewandten Stirnseite des Zylinders und/oder in der Mantelfläche des Zylinders angeordnet sein kann. Durch diesen ersten Kanal kann ein Fluid in den Zylinder strömen, um das Kolbenelement in Richtung des Meißelwerkzeugs zu bewegen. Auch diese Maßnahme dient dazu, die Prozess- und Arbeitssicherheit weiter zu erhöhen.

Der Zylinder kann auf der dem Meißelwerkzeug zugewandten Seite einen zweiten Kanal umfassen, wobei der Zylinder vorzugsweise durch den zweiten Kanal mit der ersten Staudruckkammer strömungsverbindbar ist. Auf der dem Meißelwerkzeug zugewandten Seite bedeutet im Sinne der Erfindung, dass der Kanal auf der dem Meißelwerkzeug zugewandten Stirnseite des Zylinders und/oder in der Mantelfläche des Zylinders angeordnet sein kann. Durch diesen zweiten Kanal kann ein Fluidaustausch zwischen Zylinder und erster Staudruckkammer erfolgen.

Die erste Staudruckkammer kann einen dritten Kanal umfassen, wobei die erste Staudruckkammer vorzugsweise durch den dritten Kanal mit der Fluidquelle strömungsverbindbar ist. Über den dritten Kanal kann ein Fluid von der Fluidquelle in die erste Staudruckkammer strömen. Der dritte Kanal ist vorzugsweise auf der dem Meißelwerkzeug abgewandten Seite der ersten Staudruckkammer angeordnet. Dadurch können kürzere oder kompaktere Kanalstrukturen für das Fluid erreicht werden.

Der Zylinder kann auf der dem Meißelwerkzeug abgewandten Seite einen vierten Kanal umfassen, wobei der Zylinder vorzugsweise durch den vierten Kanal mit der zweiten Staudruckkammer strömungsverbindbar ist. Über diesen vierten Kanal kann ein Fluidaustausch zwischen Zylinder und zweiter Staudruckkammer erfolgen.

Der erste, dritte und vierte Kanal können wahlweise verschließbar sein. Der erste, dritte und vierte Kanal können dabei unabhängig voneinander geöffnet oder geschlossen werden. Unter verschließbar im Sinne der Erfindung ist zu verstehen, dass ein Kanal fluiddicht verschlossen ist. Zumindest ist damit eine derartige Fluiddichtigkeit gemeint, so dass die angedachte Funktion der Abdichtung erreicht werden kann. Eine Leckage kann toleriert werden, wenn die Funktion nicht nachteilig beeinträchtigt wird.

Zum Eintreiben des Meißelwerkzeugs in den Prothesenschaftzwischenraum können der erste und zweite Kanal in einem geöffneten Zustand und der dritte und vierte Kanal in einem geschlossenen Zustand sein. Zum Eintreiben übt das Kolbenelement dabei einen Impuls auf den Impulsüberträger aus, der an das Meißelwerkzeug übertragen wird. Die Bewegung des Kolbenelements zum Eintreiben des Meißelwerkzeugs in den Prothesenschaftzwischenraum kann dabei in zwei Phasen eingeteilt werden. Während der ersten Phase wird das Fluid aus der Fluidquelle durch den ersten Kanal in den Zylinder gefördert und beschleunigt so das Kolbenelement in Richtung der dem Meißelwerkzeug zugewandten Seite bis zur Impulsübertragung auf den Impulsübertrager. Dadurch wird das Fluid im Zylinder zwischen dem Kolbenelement und der dem Meißelwerkzeug zugewandten Seite von dem sich bewegenden Kolbenelement durch den zweiten Kanal in die erste Staudruckkammer gefördert und komprimiert. Während der zweiten Phase ist die Förderung des Fluids aus der Fluidquelle unterbrochen. Weiterhin expandiert das in der ersten Staudruckkammer komprimierte Fluid und strömt durch den zweiten Kanal zurück in den Zylinder und treibt das Kolbenelement zu der dem Meißelwerkzeug abgewandten Seite des Zylinders entsprechend der Ausgangsstellung der Bewegung zum Eintreiben des Meißelwerkzeugs.

Zum Austreiben des Meißelwerkzeugs aus dem Prothesenschaftzwischenraum können der erste Kanal in einem geschlossenen Zustand und der zweite, dritte und vierte Kanal in einem geöffneten Zustand sein. Zum Austreiben übt das Kolbenelement dabei einen dem Meißelwerkzeug entgegengesetzten Impuls aus. Die Bewegung des Kolbenelements zum Austreiben des Meißelwerkzeugs aus dem Prothesenschaftzwischenraum kann dabei ebenfalls in zwei Phasen eingeteilt werden. Während der ersten Phase wird das Fluid aus der Fluidquelle durch den dritten Kanal in die erste Staudruckkammer und weiter über den zweiten Kanal in den Zylinder gefördert. Das Fluid beschleunigt das Kolbenelement in Richtung der dem Meißelwerkzeug abgewandten Seite bis zur Impulsübertragung auf den Zylinder. Das im Zylinder zwischen dem Kolbenelement und der dem Meißelwerkzeug abgewandten Seite befindliche Fluid wird von dem sich bewegenden Kolbenelement durch den vierten Kanal in die zweite Staudruckkammer gefördert und dort komprimiert. Vorzugsweise ist der vierte Kanal derart angeordnet, dass sich zwischen dem Kolbenelement und der dem Meißelwerkzeug abgewandten Seite des Zylinders kein Fluidpolster, insbesondere kein Gasdruckpolster, aufbauen kann. Dadurch kann die Impulsübertragung von dem Kolbenelement auf den Zylinder weiter verbessert werden. Während der zweiten Phase ist die Förderung des Fluids aus der Fluidquelle unterbrochen. Das in der zweiten Staudruckkammer komprimierte Fluid expandiert und strömt durch den vierten Kanal zurück in den Zylinder und treibt das Kolbenelement zu der dem Meißelwerkzeug zugewandten Seite des Zylinders entsprechend der Ausgangsstellung der Bewegung zum Austreiben des Meißelwerkzeugs.

Die Fluidquelle kann gepulste Druckstöße erzeugen, wobei die Wiederholungsfrequenz vorzugsweise im Bereich zwischen 1 und 40 Hz liegt. Besonders bevorzugt liegt die Wiederholungsfrequenz im Bereich zwischen 2 und 20 Hz. Diese gepulsten Druckstöße ermöglichen gezielte Wiederholungen des Impulses in einem kleinen Zeitfenster. Im Vergleich zu manuellen Auslösungen des chirurgischen Instruments sind dabei höhere Wiederholungsfrequenzen möglich. Durch diese Maßnahmen wird die Prozess- und Arbeitssicherheit und die Handhabbarkeit weiter verbessert.

Das Schaltelement kann als Drehgriff ausgebildet sein, wobei das Schaltelement eine Kanalstruktur aufweist, welche eine Strömungsverbindung entweder zwischen der Fluidquelle und dem Zylinder mittels des ersten Kanals oder zwischen der Fluidquelle und der ersten Staudruckkammer mittels des dritten Kanals einstellt. Der Drehgriff verbessert die Handhabbarkeit, da die Wahl des Bedieners zwischen Ein-und Austreiben mittels eines Drehgriffs erfolgt. Zur Auswahl der Schalterstellung des Drehgriffs sind dabei vorteilhafterweise nur kleine Drehungen notwendig. Ein aufwändiges Umgreifen, neu Greifen des chirurgischen Instruments oder Wechsel des Blickwinkels ist nicht notwendig. Des Weiteren können Rastmittel vorgesehen sein, um den einzelnen Schalterstellungen definierte Positionen des Drehgriffs zuzuweisen.

Das Meißelwerkzeug kann werkzeuglos austauschbar an dem Handstück angeordnet sein. Vorzugsweise wird das Meißelwerkzeug dabei mittels Bajonettverschlusses befestigt. Vorteilhaft ist dabei neben der werkzeuglosen Austauschbarkeit auch die kurze Bearbeitungszeit zur Herstellung und Lösbarkeit der mechanischen Verbindung.

An der dem Meißelwerkzeug abgewandten Seite kann ein Magnethalter angeordnet sein, wobei das Kolbenelement einen ferromagnetischen Werkstoff umfasst. Der Magnethalter ist dabei so ausgestaltet, dass er das Kolbenelement in seiner dem Meißelwerkzeug abgewandten Endposition festhalten kann. Dadurch wird einer unerwünschten Bewegung des Kolbenelements im Zylinder entgegengewirkt. Durch Fluidzufuhr aus der Fluidquelle kann die Haltekraft des Magnethalters überwunden und das Kolbenelement bewegt werden. Durch diese Maßnahme wird die Anfangsposition des Kolbenelements festgelegt, sodass eine erhöhte Prozesssicherheit erreicht werden kann.

Eine vorteilhafte Ausgestaltung der Erfindung sieht vor, dass chirurgische Instrument ein Anzeigeelement umfasst. Das Anzeigeelement ist vorzugsweise digital ausgebildet. Die darstellbaren Informationen umfassen vorzugsweise den anliegenden Fluiddruck, die eingestellte Wiederholungsrate und mögliche Fehlermeldungen. Durch diese Maßnahme erhält der Benutzer Rückmeldung über gemacht Einstellungen oder weitere nützliche Informationen, daher dient diese Maßnahme dazu die Handhabbarkeit weiter zu erhöhen.

Eine Ausgestaltung des erfindungsgemäßen chirurgischen Instruments wird nachfolgend anhand der Figuren näher erläutert. Diese zeigen, jeweils schematisch:
- Fig. 1: ein chirurgisches Instrument zur Revision von Prothesen;
- Fig. 2: das chirurgische Instrument mit Fluidquelle;
- Fig. 3: eine Schnittansicht des chirurgischen Instruments in einem Zustand zum Eintreiben des Meißelwerkzeugs;
- Fig. 4: eine erste Schnittansicht des chirurgischen Instruments in einem Zustand zum Austreiben des Meißelwerkzeugs;
- Fig. 5: eine zweite Schnittansicht des chirurgischen Instruments in einem Zustand zum Austreiben des Meißelwerkzeugs;
- Fig. 6: eine dritte Schnittansicht des chirurgischen Instruments in einem Zustand zum Austreiben des Meißelwerkzeugs.

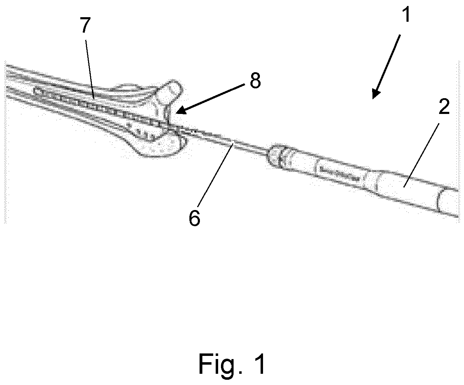

Figur 1 zeigt ein chirurgisches Instrument 1 zur Revision von Prothesen, insbesondere von Hüftgelenksendoprothesen. Das chirurgische Instrument 1 umfasst ein Handstück 2 und ein Meißelwerkzeug 6. Das Meißelwerkzeug 6 ist an einem Ende des Handstücks 2 axial beweglich gelagert angeordnet und verliersicher an dem Handstück 2 befestigt. Das Meißelwerkzeug 6 ist mittels Bajonettverschlusses befestigt und werkzeuglos austauschbar.

Zur Revision wird das Meißelwerkzeug in den der Prothese 7 zugeordneten Prothesenschaftzwischenraum 8 eingetrieben. Dabei wird die am Knochen befestigte Prothese 7 gelockert. Das erfindungsgemäße chirurgische Instrument 1 ermöglicht es aber auch, das Meißelwerkzeug 6 aus dem der Prothese 7 zugeordneten Prothesenschaftzwischenraum 8 auszutreiben. Dies ist insbesondere dann vorteilhaft, wenn das Meißelwerkzeug 6 zu tief in den Prothesenschaftzwischenraum 8 eingedrungen ist und sich dort verklemmt. Figur 1 zeigt den Revisionsvorgang, wobei das Meißelwerkzeug 6 teilweise in dem Prothesenschaftzwischenraum 8 eingetrieben ist.

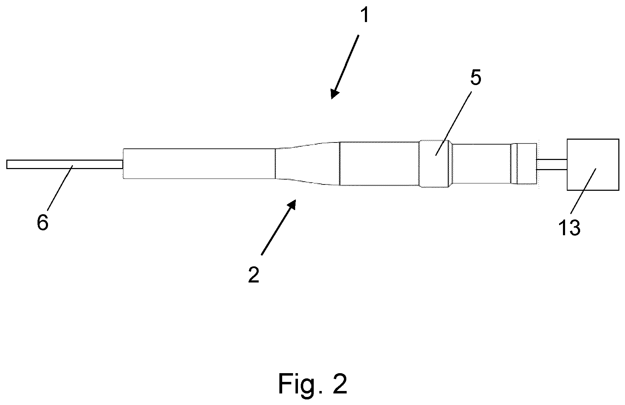

Figur 2 zeigt das chirurgische Instrument 1 gemäß Figur 1 zur Revision von Prothesen mit einer Fluidquelle 13. Vorliegend ist die Fluidquelle 13 eine Druckluftquelle. Die Druckluft kann dabei über einen Kompressor bereitgestellt werden. Alternativ ist auch denkbar, ein komprimiertes gasförmiges Medium wie Stickstoff aus einem Druckspeicher zu entnehmen.

Das chirurgische Instrument 1 umfasst ein Handstück 2, ein Schaltelement 5 und ein Meißelwerkzeug 6. Über das Schaltelement 5 kann dabei wahlweise eingestellt werden, so dass das chirurgische Instrument 1 zum Eintreiben oder zum Austreiben des Meißelwerkzeugs 6 aus dem Prothesenschaftzwischenraum 8 verwendet werden kann. Das Antriebsmittel zum Erzeugen des Impulses ist ein gasförmiges Fluid in Form von Druckluft, welche über die Fluidquelle 13 bereitgestellt wird. Die Fluidquelle 13 erzeugt gepulste Druckluftstöße, wobei die Wiederholungsfrequenz im Bereich zwischen 1 und 40 Hz liegt. Die Druckluft wird über einen Druckluftschlauch von der Fluidquelle 13 zum Handstück 2 übertragen.

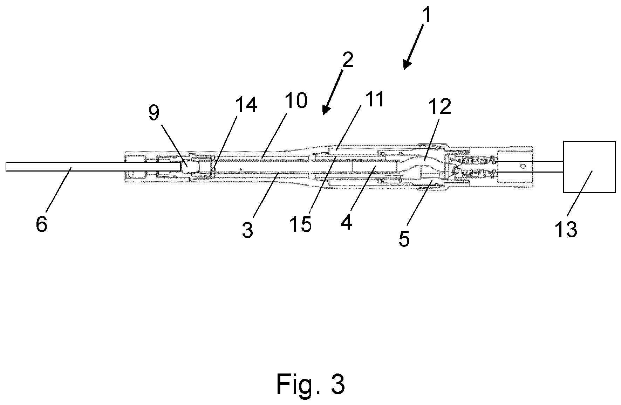

Figur 3 zeigt eine Schnittansicht des chirurgischen Instruments 1 gemäß Figur 2 in einem Zustand zum Eintreiben des Meißelwerkzeugs 6. Das chirurgische Instrument 1 umfasst ein Handstück 2, ein Schaltelement 5 und ein Meißelwerkzeug 6.

In dem Handstück 2 ist ein Zylinder 3 angeordnet, wobei der Zylinder 3 ein rohrförmiges Element ist, welches in das Handstück 2 eingesetzt ist. Dem Zylinder 3 ist ein Kolbenelement 4 zugeordnet, wobei das Kolbenelement 4 in dem Zylinder 3 axial beweglich angeordnet und mittels Druckluft in Bewegung versetzbar ist. Dabei ist das Kolbenelement 4 dazu eingerichtet, einen in Richtung des Meißelwerkzeugs 6 gerichteten Impuls zum Eintreiben des Meißelwerkzeugs 6 in den der Prothese 7 zugeordneten Prothesenschaftzwischenraum 8 zu induzieren. Weiterhin ist das Kolbenelement 4 dazu eingerichtet, einen dem Meißelwerkzeug 6 entgegengerichteten Impuls zum Austreiben des Meißelwerkzeugs 6 aus dem Prothesenschaftzwischenraum 8 zu induzieren.

Dem Zylinder 3 ist ein Impulsübertrager 9 zugeordnet. Dabei ist der Impulsübertrager 9 der dem Meißelwerkzeug 6 zugeordneten Stirnseite zugeordnet und zwischen dem Kolbenelement 4 und dem Meißelwerkzeug 6 angeordnet. Der Impulsübertrager 9 ist dazu eingerichtet, einen Impuls von dem Kolbenelement 4 auf das Meißelwerkzeug 6 zu übertragen, wobei der Impulsübertrager 9 die Stirnseite des Zylinders 3 auf der dem Meißelwerkzeug 6 zugewandten Seite abdichtet.

Der Zylinder 3 umfasst auf der dem Meißelwerkzeug 6 abgewandten Seite einen ersten Kanal 12. Dabei ist der Zylinder 3 durch den ersten Kanal 12 mit der Fluidquelle 13 strömungsverbunden.

Dem Zylinder 3 ist eine erste Staudruckkammer 10 und eine zweite Staudruckkammer 11 zugeordnet. Beide Staudruckkammern 10, 11 sind strömungsleitend mit dem Zylinder 3 verbindbar. Der Zylinder 3 umfasst auf der dem Meißelwerkzeug 6 zugewandten Seite einen zweiten Kanal 14 und ist durch den zweiten Kanal 14 mit der ersten Staudruckkammer 10 strömungsverbunden. Die erste Staudruckkammer 10 umfasst einen dritten Kanal 15 und ist durch den dritten Kanal 15 mit der Fluidquelle 13 strömungsverbindbar. In dem dargestellten Zustand ist der dritte Kanal 15 verschlossen und die erste Staudruckkammer 10 ist nicht mit der Fluidquelle 13 strömungsverbunden.

Über das Schaltelement 5 ist das Kolbenelement 4 wahlweise zum Eintreiben oder Austreiben des Meißelwerkzeugs 6 einstellbar. Das Schaltelement 5 ist dazu als Drehgriff ausgebildet und weist eine Kanalstruktur auf. Durch diese Kanalstruktur kann wahlweise eine Strömungsverbindung zwischen der Fluidquelle 13 und dem Zylinder 3 mittels des ersten Kanals 12, eine Strömungsverbindung zwischen der Fluidquelle 13 und der ersten Staudruckkammer 10 mittels des dritten Kanals 15 und eine Strömungsverbindung zwischen dem Zylinder 3 und der zweiten Staudruckkammer 11 mittels des vierten Kanals eingestellt oder unterbrochen werden. Der erste Kanal 12, der dritte Kanal 15 und der vierte Kanal 16 (hier nicht dargestellt) sind also wahlweise verschließbar.

Der erste Kanal 12 ist geöffnet, so dass eine Strömungsverbindung zwischen der Fluidquelle 13 und dem Zylinder 3 mittels des ersten Kanals 12 besteht. Der dritte Kanal 15 ist verschlossen. Der vierte Kanal 16 ist ebenfalls geschlossen und aufgrund der komplexen Geometrie der Kanalstruktur des Drehgriffs in dieser Figur nicht dargestellt.

Zum Eintreiben des Meißelwerkzeugs 6 sind der erste Kanal 12 und der zweite Kanal 14 in einem geöffneten Zustand. Der dritte Kanal 15 und der vierte Kanal sind in einem geschlossenen Zustand. Zum Eintreiben wird das Kolbenelement 4 durch den Druckluftstoß in Richtung des Impulsübertragers 9 beschleunigt und übt schließlich einen Impuls auf den Impulsüberträger 9 aus, der wiederum auf das Meißelwerkzeug 6 übertragen wird. Zur Bewegung des Kolbenelements 4 zum Eintreiben des Meißelwerkzeugs 6 wird Druckluft aus der Fluidquelle 13 durch den ersten Kanal 12 in den Zylinder 3 gefördert und beschleunigt so das Kolbenelement 4 in Richtung der dem Meißelwerkzeug 6 zugewandten Seite bis zur Impulsübertragung auf den Impulsübertrager 9. Gleichzeitig wird die Druckluft im Zylinder 3 zwischen dem Kolbenelement 4 und der dem Meißelwerkzeug 6 zugewandten Seite von dem sich bewegenden Kolbenelement 4 durch den zweiten Kanal 14 in die erste Staudruckkammer 10 gefördert und komprimiert. Sobald der von der Fluidquelle 13 ausgehende Druckluftstoß endet, expandiert die in der ersten Staudruckkammer 10 komprimierte Druckluft und strömt durch den zweiten Kanal 14 zurück in den Zylinder 3 und treibt das Kolbenelement 4 zu der dem Meißelwerkzeug 6 abgewandten Seite des Zylinders 3 zurück, entsprechend der Ausgangsstellung der Bewegung zum Eintreiben des Meißelwerkzeugs 6.

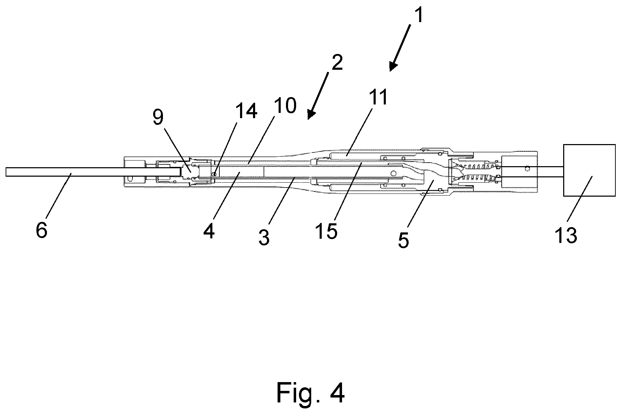

Die Figuren 4, 5 und 6 zeigen Schnittansichten des in Figur 3 gezeigten chirurgischen Instruments 1 in einem Zustand zum Austreiben des Meißelwerkzeugs 6.

Zum Austreiben des Meißelwerkzeugs 6 sind der erste Kanal 12 in einem geschlossenen Zustand und der zweite Kanal 14, der dritte Kanal 15 und der vierte Kanal 16 in einem geöffneten Zustand. Aufgrund der komplexen Geometrie der Kanalstruktur des Drehgriffs ist der erste Kanal 12 in diesen Figuren nicht dargestellt.

Der dritte Kanal 15 ist geöffnet, sodass eine Strömungsverbindung zwischen der Fluidquelle 13 und der Staudruckkammer 10 mittels des dritten Kanals 15 besteht.

Zum Austreiben übt das Kolbenelement 4 dabei einen dem Meißelwerkzeug 6 entgegengesetzten Impuls aus. Zur Bewegung des Kolbenelements 4 zum Austreiben des Meißelwerkzeugs wird ein Druckluftstoß aus der Fluidquelle 13 durch den dritten Kanal 15 in die erste Staudruckkammer 10 und weiter über den zweiten Kanal 14 in den Zylinder 3 gefördert. Die Druckluft beschleunigt das Kolbenelement 4 in Richtung der dem Meißelwerkzeug 6 abgewandten Seite bis zur Impulsübertragung auf den Zylinder 3. Die im Zylinder 3 zwischen dem Kolbenelement 4 und der dem Meißelwerkzeug 6 abgewandten Seite befindliche Druckluft wird von dem sich bewegenden Kolbenelement 4 durch den vierten Kanal 16 in die zweite Staudruckkammer 11 gefördert und dort komprimiert.

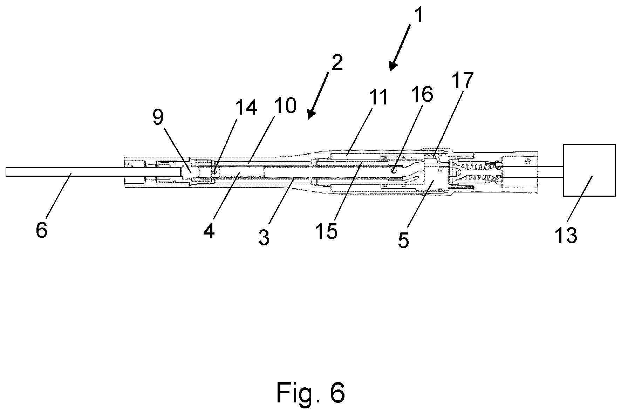

Die Druckluft, die sich zwischen der Öffnung zum vierten Kanal 16 in dem Zylinder 3 und der dem Meißelwerkzeug 6 abgewandten Seite des Zylinders 3 befindet, wird, wenn das Kolbenelement 4 die Öffnung des vierten Kanals 16 verschließt, durch den fünften Kanal 17 in die Staudruckkammer 11 gefördert. Der fünfte Kanal 17 ist beim Austreiben des Kolbenelements 4 geöffnet, so dass eine Strömungsverbindung zwischen dem Zylinder 3 und der Staudruckkammer 11 mittels des fünften Kanals 17 besteht. Aufgrund der komplexen Geometrie ist der fünfte Kanal 17 nur in Figur 6 erkennbar. Durch den fünften Kanal 17 wird beim Austreiben des Kolbenelements 4 vorteilhafterweise vermieden, dass sich zwischen dem Kolbenelement 4 und der dem Meißelwerkzeug 6 abgewandten Seite des Zylinders 3 ein Luftpolster aufbaut. Durch diese Maßnahme kann der Impulsübertrag verbessert werden. Anschließend expandiert die in der zweiten Staudruckkammer 11 komprimierte Druckluft und strömt durch den vierten Kanal 16 zurück in den Zylinder 3 und treibt das Kolbenelement 4 zu der dem Meißelwerkzeug 6 zugewandten Seite des Zylinders 3 entsprechend der Ausgangsstellung der Bewegung zum Austreiben des Meißelwerkzeugs 6.

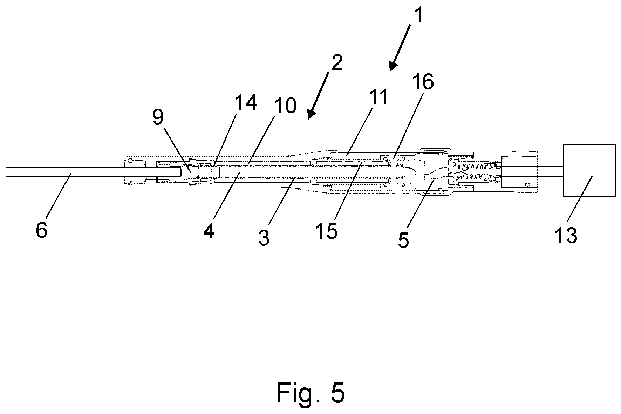

Figur 5 zeigt eine zweite Schnittansicht des in Figur 3 und Figur 4 gezeigten chirurgischen Instruments 1. Das chirurgische Instrument 1 befindet sich in einem Zustand zum Austreiben des Meißelwerkzeugs 6 gemäß Figur 4. In Figur 5 befindet sich die Schnittansicht in einer anderen Ebene.

Figur 6 zeigt eine dritte Schnittansicht des in Figur 3, Figur 4 und Figur 5 gezeigten chirurgischen Instruments 1 mit einer im Vergleich zu Figur 5 um 90° gedrehte Schnittebene. Das chirurgische Instrument 1 befindet sich in einem Zustand zum Austreiben des Meißelwerkzeugs 6 gemäß Figur 4 und Figur 5.

In den Ansichten der Figuren 5 und 6 ist jeweils die Strömungsverbindung zwischen dem Zylinder 3 und der zweiten Staudruckkammer 11 durch den vierten Kanal 16 dargestellt.

## Patentansprüche

1. Chirurgisches Instrument (1) zur Revision von Prothesen, umfassend ein Handstück (2), in welchem ein Zylinder (3) angeordnet ist, wobei dem Zylinder (3) ein Kolbenelement (4) zugeordnet ist sowie ein Schaltelement (5) und ein Meißelwerkzeug (6), wobei das Meißelwerkzeug (6) an einem Ende des Handstücks (2) axial beweglich gelagert angeordnet ist, wobei das Meißelwerkzeug (6) verliersicher am Handstück (2) befestigt ist, wobei das Kolbenelement (4) in dem Zylinder (3) axial beweglich angeordnet ist, wobei das Kolbenelement (4) mittels eines Fluids in Bewegung versetzbar ist, wobei das Kolbenelement (4) dazu eingerichtet ist, einen in Richtung des Meißelwerkzeugs (6) gerichteten Impuls zum Eintreiben des Meißelwerkzeugs (6) in einen der Prothese (7) zugeordneten Prothesenschaftzwischenraum (8) zu induzieren, wobei das Kolbenelement (4) eingerichtet ist, einen dem Meißelwerkzeug (6) entgegengerichteten Impuls zum Austreiben des Meißelwerkzeugs (6) aus dem Prothesenschaftzwischenraum (8) zu induzieren, **dadurch gekennzeichnet, dass** dem Zylinder (3) ein Impulsübertrager (9) zugeordnet ist, wobei der Impulsübertrager (9) der dem Meißelwerkzeug (6) zugeordneten Stirnseite zugeordnet ist, so dass der Impulsübertrager (9) zwischen Kolbenelement (4) und Meißelwerkzeug (6) angeordnet ist, wobei der Impulsübertrager (9) dazu eingerichtet ist, einen Impuls von dem Kolbenelement (4) auf das Meißelwerkzeug (6) zu übertragen, wobei der Impulsübertrager (9) die Stirnseite des Zylinders (3) auf der dem Meißelwerkzeug (6) zugewandten Seite abdichtet.

2. Chirurgisches Instrument (1) nach Anspruch 1, **dadurch gekennzeichnet, dass** das Kolbenelement (4) mittels des Schaltelements (5) wahlweise zum Eintreiben oder Austreiben des Meißelwerkzeugs (6) einstellbar ist.

3. Chirurgisches Instrument (1) nach Anspruch 1 oder 2, **dadurch gekennzeichnet, dass** der Zylinder (3) ein rohrförmiges Element ist, welches in das Handstück (2) eingesetzt ist.

4. Chirurgisches Instrument (1) nach einem der Ansprüche 1 bis 3, **dadurch gekennzeichnet, dass** dem Zylinder (3) eine erste Staudruckkammer (10) und eine zweite Staudruckkammer (11) zugeordnet ist, wobei die Staudruckkammern (10, 11) strömungsleitend mit dem Zylinder (3) verbindbar sind, wobei dem Zylinder (3) eine Fluidquelle (13) zugeordnet ist.

5. Chirurgisches Instrument (1) nach Anspruch 4, **dadurch gekennzeichnet, dass** der Zylinder (3) auf der dem Meißelwerkzeug (6) abgewandten Seite ein erster Kanal (12) umfasst, wobei der Zylinder (3) durch den ersten Kanal (12) mit der Fluidquelle (13) strömungsverbindbar ist.

6. Chirurgisches Instrument (1) nach Anspruch 4 oder 5, **dadurch gekennzeichnet, dass** der Zylinder (3) auf der dem Meißelwerkzeug (6) zugewandten Seite einen zweiten Kanal (14) umfasst, wobei der Zylinder (3) durch den zweiten Kanal (14) mit der ersten Staudruckkammer (10) strömungsverbindbar ist.

7. Chirurgisches Instrument (1) nach einem der Ansprüche 4 bis 6, **dadurch gekennzeichnet, dass** die erste Staudruckkammer (10) einen dritten Kanal (15) umfasst, wobei die erste Staudruckkammer (10) durch den dritten Kanal (15) mit der Fluidquelle (13) strömungsverbindbar ist.

8. Chirurgisches Instrument (1) nach einem der Ansprüche 4 bis 7, **dadurch gekennzeichnet, dass** der Zylinder (3) auf der dem Meißelwerkzeug (6) abgewandten Seite einen vierten Kanal (16) umfasst, wobei der Zylinder (3) durch den vierten Kanal (16) mit der zweiten Staudruckkammer (11) strömungsverbindbar ist.

9. Chirurgisches Instrument (1) nach Anspruch 8, **dadurch gekennzeichnet, dass** der erste, dritte und vierte Kanal (12, 15, 16) wahlweise verschließbar sind.

10. Chirurgisches Instrument (1) nach Anspruch 9, **dadurch gekennzeichnet, dass** zum Eintreiben des Meißelwerkzeugs (6) in den Prothesenschaftzwischenraum (8) der erste und zweite Kanal (12, 14) in einem geöffneten Zustand und der dritte und vierte Kanal (15, 16) in einem geschlossenen Zustand sind.

11. Chirurgisches Instrument (1) nach Anspruch 10, **dadurch gekennzeichnet, dass** zum Austreiben des Meißelwerkzeugs (6) aus dem Prothesenschaftzwischenraum (8) der erste Kanal (12) in einem geschlossenen Zustand und der zweite, dritte und vierte Kanal (14, 15, 16) in einem geöffneten Zustand sind.

12. Chirurgisches Instrument (1) nach einem der Ansprüche 4 bis 11, **dadurch gekennzeichnet, dass** die Fluidquelle (13) gepulste Druckluftstöße erzeugt, wobei die Wiederholungsfrequenz im Bereich zwischen 1 und 40 Hz liegt.

13. Chirurgisches Instrument (1) nach einem der Ansprüche 1 bis 12, **dadurch gekennzeichnet, dass** das Schaltelement (5) als Drehgriff ausgebildet ist, wobei das Schaltelement (5) eine Kanalstruktur aufweist, welche in einer ersten Position eine Strömungsverbindung zwischen der Fluidquelle (13) und dem Zylinder (3) mittels des ersten Kanals (12) herstellt und eine Strömungsverbindung zwischen der Fluidquelle (13) und der ersten Staudruckkammer (10) mittels des dritten Kanals (15) unterbricht und eine Strömungsverbindung zwischen dem Zylinder (3) und der zweiten Staudruckkammer (11) mittels des vierten Kanals (16) unterbricht, oder in einer zweiten Position eine Strömungsverbindung zwischen der Fluidquelle (13) und dem Zylinder (3) mittels des ersten Kanals (12) unterbricht und eine Strömungsverbindung zwischen der Fluidquelle (13) und der ersten Staudruckkammer (10) mittels des dritten Kanals (15) herstellt und eine Strömungsverbindung zwischen dem Zylinder (3) und der zweiten Staudruckkammer (11) mittels des vierten Kanals (16) herstellt.

## Claims

1. A surgical instrument (1) for the revision of prostheses, comprising a handpiece (2) in which a cylinder (3) is arranged, wherein a piston element (4) is associated with the cylinder (3), as well as a switching element (5) and a chisel tool (6), wherein the chisel tool (6) is arranged axially movably mounted at one end of the handpiece (2), wherein the chisel tool (6) is fastened to the handpiece (2) in a loss-proof manner, wherein the piston element (4) is axially movably arranged in the cylinder (3), wherein the piston element (4) can be set in motion by means of a fluid, wherein the piston element (4) is configured to induce a pulse directed in the direction of the chisel tool (6) for driving the chisel tool (6) into a prosthesis stem interspace (8) associated with the prosthesis (7), wherein the piston element (4) is configured to induce a pulse directed opposite to the chisel tool (6) for driving out the chisel tool (6) from the prosthesis stem interspace (8), **characterized in that** a pulse transmitter (9) is associated with the cylinder (3), wherein the pulse transmitter (9) is associated with the end face associated with the chisel tool (6), so that the pulse transmitter (9) is arranged between the piston element (4) and the chisel tool (6), wherein the pulse transmitter (9) is configured to transmit a pulse from the piston element (4) to the chisel tool (6), wherein the pulse transmitter (9) seals the end face of the cylinder (3) on the side facing the chisel tool (6).

2. The surgical instrument (1) according to claim 1, **characterized in that** the piston element (4) is selectively configurable by means of the switching element (5) for driving in or driving out the chisel tool (6).

3. The surgical instrument (1) according to claim 1 or 2, **characterized in that** the cylinder (3) is a tubular element which is inserted into the handpiece (2).

4. The surgical instrument (1) according to any of claims 1 to 3, **characterized in that** a first dynamic pressure chamber (10) and a second dynamic pressure chamber (11) are associated with the cylinder (3), wherein the dynamic pressure chambers (10, 11) can be connected to the cylinder (3) in a flow-conducting manner, wherein a fluid source (13) is associated with the cylinder (3).

5. The surgical instrument (1) according to claim 4, **characterized in that** the cylinder (3) comprises a first channel (12) on the side facing away from the chisel tool (6), wherein the cylinder (3) is flow-connectable to the fluid source (13) through the first channel (12).

6. The surgical instrument (1) according to claim 4 or 5, **characterized in that** the cylinder (3) comprises a second channel (14) on the side facing the chisel tool (6), wherein the cylinder (3) is flow-connectable to the first dynamic pressure chamber (10) through the second channel (14).

7. The surgical instrument (1) according to any of claims 4 to 6, **characterized in that** the first dynamic pressure chamber (10) comprises a third channel (15), wherein the first dynamic pressure chamber (10) is flow-connectable to the fluid source (13) through the third channel (15).

8. The surgical instrument (1) according to any of claims 4 to 7, **characterized in that** the cylinder (3) comprises a fourth channel (16) on the side facing away from the chisel tool (6), wherein the cylinder (3) is flow-connectable to the second dynamic pressure chamber (11) through the fourth channel (16).

9. The surgical instrument (1) according to claim 8, **characterized in that** the first, third and fourth channels (12, 15, 16) are selectively closable.

10. The surgical instrument (1) according to claim 9, **characterized in that**, for driving the chisel tool (6) into the prosthesis stem interspace (8), the first and second channels (12, 14) are in an open state and the third and fourth channels (15, 16) are in a closed state.

11. The surgical instrument (1) according to claim 10, **characterized in that**, for driving out the chisel tool (6) from the prosthesis stem interspace (8), the first channel (12) is in a closed state and the second, third and fourth channels (14, 15, 16) are in an open state.

12. The surgical instrument (1) according to any of claims 4 to 11, **characterized in that** the fluid source (13) generates pulsed compressed air bursts, wherein the repetition frequency is in the range between 1 and 40 Hz.

13. The surgical instrument (1) according to any of claims 1 to 12, **characterized in that** the switching element (5) is designed as a rotary handle, wherein the switching element (5) has a channel structure, which, in a first position, establishes a flow connection between the fluid source (13) and the cylinder (3) by means of the first channel (12) and interrupts a flow connection between the fluid source (13) and the first dynamic pressure chamber (10) by means of the third channel (15) and interrupts a flow connection between the cylinder (3) and the second dynamic pressure chamber (11) by means of the fourth channel (16), or, in a second position, interrupts a flow connection between the fluid source (13) and the cylinder (3) by means of the first channel (12) and establishes a flow connection between the fluid source (13) and the first dynamic pressure chamber (10) by means of the third channel (15) and establishes a flow connection between the cylinder (3) and the second dynamic pressure chamber (11) by means of the fourth channel (16).

## Revendications

1. Instrument chirurgical (1) pour la révision de prothèses, comprenant une pièce à main (2) dans laquelle est disposé un cylindre (3), dans lequel un élément formant piston (4) est associé au cylindre (3) ainsi qu'un élément de commutation (5) et un outil de burinage (6), dans lequel l'outil de burinage (6) est disposé de manière à être mobile axialement à une extrémité de la pièce à main (2), dans lequel l'outil de burinage (6) est fixé à la pièce à main (2) de manière à ne pas être perdu, dans lequel l'élément formant piston (4) est disposé dans le cylindre (3) de manière à être mobile axialement, dans lequel l'élément formant piston (4) peut être mis en déplacement au moyen d'un fluide, dans lequel l'élément formant piston (4) est conçu pour induire une impulsion dirigée dans le sens de l'outil de burinage (6) pour l'enfoncement de l'outil de burinage (6) dans un espace intermédiaire de tige de prothèse (8) associé à la prothèse (7), dans lequel l'élément formant piston (4) est conçu pour induire une impulsion dirigée dans le sens inverse à celui de l'outil de burinage (6) pour l'expulsion de l'outil de burinage (6) hors de l'espace intermédiaire de tige de prothèse (8), **caractérisé en ce qu'**un transmetteur d'impulsions (9) est associé au cylindre (3), dans lequel le transmetteur d'impulsions (9) est associé au côté frontal associé à l'outil de burinage (6), de sorte que le transmetteur d'impulsions (9) est disposé entre l'élément formant piston (4) et l'outil de burinage (6), dans lequel le transmetteur d'impulsions (9) est conçu pour transmettre une impulsion de l'élément formant piston (4) à l'outil de burinage (6), dans lequel le transmetteur d'impulsions (9) rend étanche le côté frontal du cylindre (3) sur le côté tourné vers l'outil de burinage (6).

2. Instrument chirurgical (1) selon la revendication 1, **caractérisé en ce que** l'élément formant piston (4) peut être réglé, au moyen de l'élément de commutation (5), au choix pour l'enfoncement ou l'expulsion de l'outil de burinage (6).

3. Instrument chirurgical (1) selon la revendication 1 ou 2, **caractérisé en ce que** le cylindre (3) est un élément tubulaire qui est inséré dans la pièce à main (2).

4. Instrument chirurgical (1) selon l'une des revendications 1 à 3, **caractérisé en ce qu'**une première chambre de pression dynamique (10) et une seconde chambre de pression dynamique (11) sont associées au cylindre (3), dans lequel les chambres de pression dynamique (10, 11) peuvent être reliées au cylindre (3) d'une manière conductrice d'écoulement, dans lequel une source de fluide (13) est associée au cylindre (3).

5. Instrument chirurgical (1) selon la revendication 4, **caractérisé en ce que** le cylindre (3) comprend un premier canal (12) sur le côté opposé à l'outil de burinage (6), dans lequel le cylindre (3) peut être relié par écoulement à la source de fluide (13) au moyen du premier canal (12).

6. Instrument chirurgical (1) selon la revendication 4 ou 5, **caractérisé en ce que** le cylindre (3) comprend un deuxième canal (14) sur le côté tourné vers l'outil de burinage (6), dans lequel le cylindre (3) peut être relié par écoulement à la première chambre de pression dynamique (10) au moyen du deuxième canal (14).

7. Instrument chirurgical (1) selon l'une des revendications 4 à 6, **caractérisé en ce que** la première chambre de pression dynamique (10) comprend un troisième canal (15), dans lequel la première chambre de pression dynamique (10) peut être reliée par écoulement à la source de fluide (13) au moyen du troisième canal (15).

8. Instrument chirurgical (1) selon l'une des revendications 4 à 7, **caractérisé en ce que** le cylindre (3) comprend un quatrième canal (16) sur le côté opposé à l'outil de burinage (6), dans lequel le cylindre (3) peut être relié par écoulement à la seconde chambre de pression dynamique (11) au moyen du quatrième canal (16).

9. Instrument chirurgical (1) selon la revendication 8, **caractérisé en ce que** les premier, troisième et quatrième canaux (12, 15, 16) peuvent être obturés au choix.

10. Instrument chirurgical (1) selon la revendication 9, **caractérisé en ce que**, pour l'enfoncement de l'outil de burinage (6) dans l'espace intermédiaire de tige de prothèse (8), les premier et deuxième canaux (12, 14) sont dans un état ouvert et les troisième et quatrième canaux (15, 16) sont dans un état fermé.

11. Instrument chirurgical (1) selon la revendication 10, **caractérisé en ce que**, pour l'expulsion de l'outil de burinage (6) hors de l'espace intermédiaire de tige de prothèse (8), le premier canal (12) est dans un état fermé et les deuxième, troisième et quatrième canaux (14, 15, 16) sont dans un état ouvert.

12. Instrument chirurgical (1) selon l'une des revendications 4 à 11, **caractérisé en ce que** la source de fluide (13) génère des poussées d'air comprimé pulsées, dans lequel la fréquence de répétition est comprise dans la plage comprise entre 1 et 40 Hz.

13. Instrument chirurgical (1) selon l'une des revendications 1 à 12, **caractérisé en ce que** l'élément de commutation (5) est conçu comme une poignée rotative, dans lequel l'élément de commutation (5) présente une structure de canal qui, dans une première position, établit une liaison par écoulement entre la source de fluide (13) et le cylindre (3) au moyen du premier canal (12) et interrompt une liaison par écoulement entre la source de fluide (13) et la première chambre de pression dynamique (10) au moyen du troisième canal (15) et interrompt une liaison par écoulement entre le cylindre (3) et la seconde chambre de pression dynamique (11) au moyen du quatrième canal (16), ou qui, dans une seconde position, interrompt une liaison par écoulement entre la source de fluide (13) et le cylindre (3) au moyen du premier canal (12) et établit une liaison par écoulement entre la source de fluide (13) et la première chambre de pression dynamique (10) au moyen du troisième canal (15) et établit une liaison par écoulement entre le cylindre (3) et la seconde chambre de pression dynamique (11) au moyen du quatrième canal (16).
